Europäisches Patentamt

European Patent Office

Office européen des brevets

(11) Publication number: **0 130 160**
**B1**

(12) **EUROPEAN PATENT SPECIFICATION**

(45) Date of publication of patent specification: **07.01.88**

(21) Application number: **84850176.3**

(22) Date of filing: **12.06.84**

(51) Int. Cl.⁴: **B 01 J 13/02,** A 61 K 9/50,
A 61 K 9/14, B 01 J 19/00

(54) **Small particle formation.**

(30) Priority: **22.06.83 US 506598**

(43) Date of publication of application:
**02.01.85 Bulletin 85/01**

(45) Publication of the grant of the patent:
**07.01.88 Bulletin 88/01**

(84) Designated Contracting States:
**AT BE CH DE FR GB IT LI LU NL SE**

(56) References cited:
**EP-A-0 116 182**
**DE-A-3 013 839**
**FR-A-2 106 033**
**FR-A-2 160 932**

The file contains technical information
submitted after the application was filed and
not included in this specification

(73) Proprietor: **THE OHIO STATE UNIVERSITY**
**RESEARCH FOUNDATION**
**1314 Kinnear Road**
**Columbus Ohio 43212 (US)**

(72) Inventor: **Frank, Sylvan Gerald**
**1910 Wyandotte Road**
**Columbus Ohio 43212 (US)**
Inventor: **Brodin, Arne Folke**
**Gillestigen 29**
**S-151 52 Södertälje (SE)**
Inventor: **Chen, Chih-Ming James**
**414 Deerfield Road, Apt 4**
**East Syracuse New York 13057 (US)**
Inventor: **Shrivastava, Ratnesh**
**500 W. 12th Avenue**
**Columbus Ohio 42210 (US)**

(74) Representative: **Wurm, Bengt Runio et al**
**Patent and Trade Mark Department AB ASTRA**
**S-151 85 Södertälje (SE)**

Courier Press, Leamington Spa, England.

## Description

The present invention is concerned with the formation of small particles of organic compounds upon precipitation at a selected temperature in the presence of a surfactant mixture, induced by pH change from a first pH at which their solubility in water is greater to a second pH at which it is lower. In this application, a small particle refers to a particle size of less than 2 µm. The object of the invention is to provide a process for the formation of small particles of organic compounds especially pharmaceutically active compounds.

The rate and extent of absorption of a pharmaceutically active compound by a patient is dependent on the particle size of the compound. The administration of pharmaceutically active compounds having smaller particles makes it possible to give a reduced dosage at lower cost and results in fewer side effects.

Background of the invention

From a pharmaceutical point of view, the smaller the particle size of a relatively insoluble drug, the greater is its rate of solution and as a rule, the greater is its bioavailability, (J. H. Fincher, J. Pharm. Sci., 57, 1825 (1968). To this end, small particles are conventionally formed by mechanically subdivision of bulk matter or by aggregation of small molecules or ions, (D. J. Shaw, "Introduction to Colloid and Surface Chemistry" 3rd Edition, Butterworths, London, 1980, Chapter 1). The initial rate of nucleation depends on the relative degree of supersaturation of the solute, while the rate of particle growth depends on several factors, including the amount of material available, the viscosity of the medium, adsorption of impurities onto the particle surface and particle-particle interaction, (D. J. Shaw, "Introduction to Colloid and Surface Chemistry", 3rd Edition, Butterworths, London, 1980, Chapter 1). The coacervation of ionic dyes with ionic surfactants has been reported, (S. P. Moulik, S. Ghosh and A. R. Das, Colloid & Polymer Sci., 257, 645 (1979); B. W. Barry and G. F. J. Russell, J. Pharm. Sci., 61, 502 (1972)).

From the specification of DE—A—3 031 839 it is known to use a high molecular weight substance to form a disperse system containing a drug in the form of finely divided particles.

Summary of the invention

A method has now been found which is useful for forming small particles of weakly acidic and weakly basic organic compounds upon precipitation at a selected temperature in the presence of a surfactant mixture, induced by pH change from a first pH at which their solubility in water is greater to a second pH at which it is lower. The method comprises the steps of

(a) dissolving the compound in water.

When said compound is weakly acidic, it is dissolved in the presence of sufficient base to raise the pH of the solution to said first pH, and above the pKa of the compound, preferably about 2 pH units, together with an anionic surfactant which maintains its ionic condition between said first pH and said second pH and an amphoteric surfactant which is anionic at said first pH and whose cationic nature increases as the pH is changed from the first pH to said second pH. When said compound is weakly basic, it is dissolved in the presence of sufficient acid to lower the pH to said first pH and below the pKa of said compound, preferably about 2 pH units, together wihh a cationic surfactant which maintains its ionic condition between said first pH and said second pH, and an amphoteric surfactant which is cationic at said first pH and whose anionic nature increases as the pH is changed from the first pH to said second pH.

(b) stirring and titrating the solution, with a suitable acid titrant (if the starting solution is basic) or a suitable basic titrant (if the starting solution is acidic) in an amount effective to alter the pH from said first pH to said second pH and thereby cause the concurrent formation of a coacervate of the surfactants, and precipitation of the compound as small particles. The said second pH may be about 2 pH units above or below the pKa of the compound to precipitate the free acid, free base or the salt forms of the compound.

It is believed that as the pH of the solution changes, the compound's solubility is altered and a coacervate forms between the anionic or cationic surfactant (as the case may be) and the amphoteric surfactant simultaneously with the precipitation of the compound.

Detailed description of the invention

This process is preferably used to form small particles of organic compounds whose solubility in water is greater at a first pH than at a second pH. Such compounds are commonly found in the pharmaceutical industry and are preferably used in small-particle form as explained above.

Depending on the protolytic properties of such an organic compound it can be dissolved in either an alkaline (weakly acidic compound) or acidic solution (weakly basic compound) and precipitated by the subsequent titration with either an acid or alkaline titrant, respectively. The starting pH should preferably be 2 pH units above the pKa of a weakly acid compound and preferably 2 pH units below the pKa of a weakly basic compound.

Suitable pharmaceutically active compounds which can be used in this process are, for example, sulfadiazine, lidocaine, salicyclic acid, felodipine, sulbactam pivoxil, chlorzoxazone, theophylline and erythromycin. Suitable amphoteric surfactants which change ionic character between the first and second pH are, for example, surfactants derived from fatty imidazolines (Miranols®), particularly monocarboxylated compounds, such as Miranol® SM, which is a clear, aqueous, amphoteric solution, derived from 99% capric acid; the surfactant is a monocarboxylated derivative of a capryl imidazoline.

2

**0 130 160**

Other suitable amphoteric surfactants are, for example, betaines, such as cocamidopropyl betaine, lauramidopropyl betaine; amino acid amphoterics such as disodium lauriminodipropionate; and imidazolines derived amphoterics such as Miranol® SM and other members of these general classes.

Suitable anionic surfactants which maintain their ionic condition between the first and second pH of the weakly acidic organic compounds are, the common salts of natural and synthetic organic carboxylates, sulfonates and sulfates, such as for example, sodium or potassium stearates, sodium lauryl sulfate, sodium or potassium alkyl sulfates having alkyl groups with 8—18 carbon atoms and dialkyl sodium sulfosuccinates having alkyl groups with 6—8 carbon atoms.

Suitable cationic surfactants which maintain their ionic condition between the first and second pH of the weakly basic organic compounds are common surface-active derivatives of ammonium and various amines, for example, alkyltrimethylammonium halides containing alkyl groups with 11—18 carbon atoms, alkylpyridinium halides containing alkyl groups with 8—18 carbon atoms, benzylalkyldimethylammonium halides containing alkyl groups with 8—18 carbon atoms, and alkyldimethylethylammonium halides containing alkyl groups with 8—18 carbon atoms.

A suitable molar ratio of the pharmaceutically active compound to amphoteric surfactant and the anionic or cationic surfactant is for example 0.15:1:1 to 4.4:1:1, up to the maximum solubilizing capacity for a particular system.

The alkaline solution used to dissolve the weakly acidic compounds can be, for example, sodium hydroxide or potassium hydroxide solutions. The alkaline solution should be about 0.05—5.0 N, preferably 0.05 N or 0.1 N in order to obtain a pH preferably 2 units above the pKa of the compound. For dissolving the weakly basic compounds, the acidic solutions should be 0.05—5.0 N, preferably 0.05 N or 0.1 N in order to obtain a pH preferably 2 units below the pKa of the compound.

The titrations are performed with stirring using a suitable acid titrant, such as hydrochloric acid to reduce the pH of the solution to anywhere below pH 9 to pH 1.5, or in the case of an alkaline titrant, to a pH anywhere above pH 2 up to pH 12 and to cause the concurrent formation of a coacervate of the surfactants and precipitation of the compounds as small particles.

The molarity of the acid titrant should be in the range 0.05—5.0 N, preferably 0.1 N or 1.0 N, and that of the alkaline titrant should be in the range of 0.05—5.0 N, preferably 0.1 N or 1.0 N. Higher normalities can be used as well to obtain the desired pH.

The titration should be preformed within the temperature range of 0—50°C, usually at about 22°C.

While the invention is described with particular reference to pharmaceutical manufacture, it should be understood that the basic principles are not so limited. Obviously when applied to pharmaceuticals, the surfactants, acids and bases used should not leave pharmaceutically objectionable residues.

Example 1

Appropriate molar amounts of sulfadiazine, sodium lauryl sulfate and Miranol SM (42—44% solids by weight) as indicated in Table 1 were dissolved in sodium hydroxide solution, 0.05 N NaOH, when 0.044 M or 0.0044 M sulfadiazine was used or 0.1 N, for 0.088 M sulfadiazine. The solutions were then stirred at constant speed with a magnetic stirrer and sulfadiazine was precipitated upon dropwise titration of the solutions with 1.0 N hydrochloric acid solution.

The effect of several different composite ratios of sulfadiazine, Miranol SM and sodium lauryl sulfate on the precipitation of sulfadiazine is summarized in Table 1. As a general rule, precipitation of the sulfadiazine began when the pH reached 8.5—8.6, as indicated by increasing turbidity. Samples 1—5 represent the process of this invention while Sample A does not.

TABLE 1

Precipitation of sulfadiazine upon acidification of alkaline solutions containing surfactants

| Sample | Sulfadiazine: Miranol Molar ratio | SM: Sodium lauryl sulfate Concentration ratio | pH of appearance of turbidity | Observations of precipitate upon acidification to pH 4. |
|---|---|---|---|---|
| 1 | 1:1:1 | 0.044M:0.045M:0.045M | 8.5—8.6 | Rod-shaped particles and needles. 1—12 µm Oval-shaped particles <1 µm Droplets of coacervate phase entrapping some particles |
| 2 | 1:2:2 | 0.044M:0.09M:0.09M | 8.5—8.6 | Rod- and oval-shaped particles <1 µm. Larger rods up to 4 µm Droplets of coacervate phase entrapping some particles |
| 3 | 2:2:2 | 0.088M:0.09M:0.09M | ~8.9 | small oval- or rod-shaped particles 1 µm |
| 4 | 2:1:1 | 0.088M:0.045M:0.045M | ~8.9 | small oval- or rod-shaped particles <1 µm |
| 5 | 4.4:1:1 | 0.088M:0.02M:0.02M | ~8.9 | small oval- or rod-shaped particles <1 µm |
| A | 0.1:1:1 | 0.0044M:0.045M:0.045M | 6.8—7.0 | large needle shaped crystals of sulfadiazine (10—30 µm) |

**Claims**

1. A process for the forming of small particles of a weakly acidic organic compound whose solubility to water is greater at a first pH than at a second pH characterized by

(a) dissolving said compound in water in the presence of sufficient base to raise the pH to said first pH and above the pKa of the compound preferably about 2 pH units, together with an anionic surfactant which maintains its ionic condition between the first and second pH and an amphoteric surfactant whose cationic nature increases from the first pH to said second pH; and

(b) stirring and titrating the solution, with a titrant effective to reduce the pH of said solution to said second pH to cause the concurrent formation of a coacervate of the anionic and amphoteric surfactants, and precipitation of the compound as small particles.

2. A process for the forming of small particles of a weakly basic organic compound whose solubility in water is greater at a first pH than at a second pH characterized by

(a) dissolving said compound in water in the presence of sufficient acid to lower the pH to said first pH and below the pKa of the compound preferably about 2 pH units, together with a cationic surfactant which maintains its ionic condition between the first and second pH and an amphoteric surfactant whose anionic nature increases from the first pH to said second pH; and

(b) stirring and titrating the solution, with a titrant effective to raise the pH of said solution to said second pH to cause the concurrent formation of a coacervate of the cationic and amphoteric surfactants, and precipitation of the compound as small particles.

3. A process according to claims 1 or 2, wherein the compound is pharmaceutically active.

4. A process according to claim 3, wherein the pharmaceutically active compound is selected from the group consisting of sulfadiazine, lidocaine, salicylic acid, felodipine, sulbactam pivoxil, chlorzoxazone, theophylline and erythromycin.

5. The process according to claim 1 or 2 wherein the amphoteric surfactant is selected from the group consisting of imidazoline derived amphoterics, betaines and amino acid amphoterics.

6. A process according to claim 5, wherein the amphoteric surfactant is selected from the group consisting of cocamidoprofyl betaine, lauramidopropyl betaine, disodium and lauriminodipropionate.

7. A process according to claim 1, wherein the anionic surfactant is selected from the group consisting of sodium lauryl sulfate, sodium alkyl sulfates having alkyl groups containing 8—18 carbon atoms and dialkyl sodium sulfosuccinates having alkyl groups containing 6—8 carbon atoms.

8. A process according to claim 2, wherein the cationic surfactant is selected from the group consisting of alkyltrimethylammonium halides containing alkyl groups containing 11—18 carbon atoms, alkylpyridinium halides containing alkyl groups containing 8—18 carbon atoms, benzylalkyldimethylammonium halides containing alkyl groups with 8—18 carbon atoms and alkyldimethylethylammonium halides containing alkyl groups containing 8—18 carbon atoms.

9. A process according to claims 1 or 2, wherein the ratio of compound to amphoteric surfactant and the cationic or anionic surfactant is about 0.15:1:1 to 4.4:1:1, and up to the maximum solubilizing capacity for a particular system.

**Patentansprüche**

1. Verfahren zur Bildung kleiner Teilchen einer schwach sauren organischen Verbindung, deren Löslichkeit in Wasser bei einem ersten pH-Wert größer ist als bei einem zweiten pH-Wert, gekennzeichnet durch

(a) Lösen der Verbindung in Wasser in Anwesenheit von genügend Base zur Anhebung des pH-Wertes auf den ersten pH-Wert und über den pKa der Verbindung, vorzugsweise etwa 2 pH-Einheiten, zusammen mit einem anionischen grenzflächenaktiven Agens, das seinen ionischen Zustand zwischen dem ersten und dem zweiten pH-Wert beibehält, und einem amphoteren grenzflächenaktiven Agens, dessen kationische Natur vom ersten pH-Wert zum zweiten pH-Wert zunimmt; und

(b) Rühren und Titrieren der Lösung mit einem Titrans, welches zur Reduktion des pH-Werts der Lösung auf den zweiten pH-Wert wirksam ist, um die gleichzeitige Bildung eines Koazervats der anionischen und amphoteren grenzflächenaktiven Agentien und die Ausfällung der Verbindung als kleine Teilchen zu bewirken.

2. Verfahren zur Bildung kleiner Teilchen einer schwach basischen organischen Verbindung, deren Löslichkeit in Wasser bei einem ersten pH-Wert größer ist als bei einem zweiten pH-Wert, gekennzeichnet durch

(a) Lösen der Verbindung in Wasser in Anwesenheit von genügend Säure zur Senkung des pH-Wertes auf den ersten pH-Wert und unter den pKa der Verbindung, vorzugsweise etwa 2 pH-Einheiten, zusammen mit einem kationischen grenzflächenaktiven Agens, das seinen ionischen Zustand zwischen dem ersten und dem zweiten pH-Wert beibehält, und einem amphoteren grenzflächenaktiven Agens, dessen anionische Natur vom ersten pH-Wert auf den zweiten pH-Wert zunimmt; und

(b) Rühren und Titrieren der Lösung mit einem Titrans, welches zur Anhebung des pH-Werts der Lösung auf den zweiten pH-Wert wirksam ist, um die gleichzeitige Bildung eines Koazervats der kationischen und amphoteren grenzflächenaktiven Agentein und die Ausfällung der Verbindung als kleine Teilchen zu bewirken.

3. Verfahren nach den Ansprüchen 1 oder 2, worin die Verbindung pharmazeutisch aktiv ist.

4. Verfahren nach Anspruch 3, worin die pharmazeutisch aktive Verbindung ausgewählt ist aus der Gruppe bestehend aus Sulfadiazin, Lidocain, Salicylsäure, Felodipin, Sulbactam-pivoxil, Chlorzoxazon, Theophyllin und Erythromycin.

5. Verfahren nach Anspruch 1 oder 2, worin das amphotere grenzflächenaktive Agens ausgewählt ist aus der Gruppe bestehend aus von Imidazolin stammenden Amphoterica, Betainen und Aminosäureamphoterica.

6. Verfahren nach Anspruch 5, worin das amphotere grenzflächenaktive Agens ausgewählt ist aus der Gruppe bestehend aus Cocamidoprofylbetain, Lauramidopropylbetain, Dinatrium and Lauriminodipropionat.

7. Verfahren nach Anspruch 1, worin das anionische grenzflächenaktive Agens ausgewählt ist aus der Gruppe bestehend aus Natriumlaurylsulfat, Natriumalkylsulfaten mit Alkylgruppen, die 8—18 Kohlenstoffatome enthalten und Dialkylnatriumsulfosuccinaten mit Alkylgruppen, die 6—8 Kohlenstoffatome enthalten.

8. Verfahren nach Anspruch 2, worin das kationische grenzflächenaktive Agens ausgewählt ist aus der Gruppe bestehend aus Alkyltrimethylammoniumhalogeniden mit Alkylgruppen, die 11—18 Kohlenstoffatome enthalten, Alkylpyridiniumhalogeniden mit Alkylgruppen, die 8—18 Kohlenstoffatome enthalten, Benzylalkyldimethylammoniumhalogeniden mit Alkylgruppen mit 8—18 Kohlenstoffatomen und Alkyldimethyläthylammoniumhalogeniden mit Alkylgruppen, die 8—18 Kohlenstoffatome enthalten.

9. Verfahren nach den Ansprüchen 1 oder 2, worin das Verhältnis der Verbindung zum amphoteren grenzflächenaktiven Agens und dem kationischen oder anionischen grenzflächenaktiven Agens etwa 0,15:1:1 bis 4,4:1:1 und bis zur maximalen Solubilisierungskapazität für ein bestimmtes Systems beträgt.

**Revendications**

1. Procédé pour former des particles fines d'un composé organique faiblement acide, dont la solubilité dans l'eau est supérieure à un premier pH à la solubilité à un second pH, procédé caractérisé en ce que

(a) on dissout ledit composé dans de l'eau en présence de suffisamment de base pour élever le pH audit premier pH et au dessus du pKa du composé de préférence d'environ 2 unités de pH, avec un tensio-actif anionique qui maintient son état ionique entre les premier et second pH et avec un tensio-actif amphotère dont la nature cationique augmente du premier pH audit second pH, et

(b) on agite et titre la solution, avec un agent de titrage ou réactif capable de réduire le pH de ladite solution audit second pH pour provoquer la formation simultanée d'un coacervat des tensio-actifs anionique et amphotère, et la précipitation du composé sous forme de particules fines.

2. Procédé pour former des particles fines d'un composé organique faiblement basique, dont la solubilité dans l'eau est supérieure à un premier pH à la solubilité à un second pH, procédé caractérisé en ce que

(a) on dissout ledit composé dans de l'eau en présence de suffisamment d'acide pour abaisser le pH audit premier pH et au dessous du pKa du composé de préférence d'environ 2 unités de pH, avec un tensio-actif cationique qui maintient son état ionique entre les premier et second pH et avec un tensio-actif amphotère dont la nature anionique augmente du premier pH audit second pH; et

(b) on agite et titre la solution, avec un agent de titrage ou réactif capable d'élever le pH de ladite solution audit second pH pour provoquer la formation simultanée d'un coacervat des tensio-actifs cationique et amphotère, et la précipitation du composé sous forme de particules fines.

3. Procédé selon la revendication 1 ou 2, dans lequel le composé est pharmaceutiquement actif.

4. Procédé selon la revendication 3, dans lequel la composé pharmaceutiquement actif est choisi parmi la sulfadiazine, la lidocaïne, l'acide salicylique, la félodipine, le sulbactam pivoxil, la chlorozoxazone, la théophylline et l'érythromycine.

5. Procédé selon la revendication 1 ou 2, dans lequel le tensioactif amphotère est choisi parmi les amphotères dérivés de l'imidazoline, les bétaïnes et les amino-acides amphotères.

6. Procédé selon la revendication 5, dans lequel le tensio-actif amphotère est choisi parmi de la coco-amidopropyl bétaïne, de la lauramidopropyl bétaïne et du lauriminodipropionate disodique.

7. Procédé selon la revendication 1, dans lequel le tensio-actif anionique est choisiparmi du lauryl sulfate de sodium, des alkyl sulfates de sodium ayant des groupes alkyles contenant 8 à 18 atomes de carbone et des dialkyl sulfosuccinates de sodium ayant des groupes alkyles contenant 6 à 8 atomes de carbone.

8. Procédé selon la revendication 2, dans lequel le tensio-actif cationique est choisi parmi des halgénures d'alkyltriméthylammonium contenant des groupes alkyles ayant 11 à 18 atomes de carbone, des halogènures d'alkylpyridinium contenant des groupes alkyles ayant 8 à 18 atomes de carbone et des halogènures d'alkyldiméthyléthylammonium contenant des groupes alkyles ayant de 8 à 18 atomes de carbone.

9. Procédé selon la revendication 1 ou 2, dans lequel le rapport entre le composé et le tensio-actif amphotère et le tensio-actif cationique ou anioànique est d'environ 0,15:1:1 à 4,4:1:1, et jusqu'à la capacité de solubilisation maximale pour un système particulier.